# EUROPEAN PATENT APPLICATION

(11) **EP 2 233 107 A1**
(43) Date of publication of application: **29.09.2010**
(21) Application number: 08718430.5
(22) Date of filing: 18.01.2008
(51) Int. Cl.: A61C 8/00

(54) **POST-EXTRACTION DENTAL IMPLANT**

(30) Priority: 07.12.2007 ES 200703256
(71) Applicant: Garcia Saban, Juan Carlos, 08211 Castellar del Valles, Barcelona (ES); Garcia Saban, Francisco Javier, 08211 Castellar del Valles, Barcelona (ES); Garcia Saban, Miguel Angel, 08211 Castellar del Valles, Barcelona (ES)
(72) Inventor: Garcia Saban, Juan Carlos, 08211 Castellar del Valles, Barcelona (ES); Garcia Saban, Francisco Javier, 08211 Castellar del Valles, Barcelona (ES); Garcia Saban, Miguel Angel, 08211 Castellar del Valles, Barcelona (ES)
(74) Representative: Carpintero Lopez, Francisco
(86) International application number: PCT/ES2008/000029
(87) International publication number: WO 2009/071712

(57) **Abstract**

The invention relates to dental implants of the type used immediately after a tooth is extracted and intended to be placed in the alveolus after the extraction, the purpose of which is to increase both the long-term and the short-term stability of the implantation, and which basically comprises a conical body on which there is located a thread (1) allowing the screwing to the cortical area of the bone and another additional self-tapping thread or micro-thread (2) of a smaller size than the thread (1) and intercalated between it, as well as means for fixing the prosthetic elements.

## Description

### Object of the Invention

As indicated in the title, the present invention patent application consists of a dental implant of the type of those used immediately after a tooth is extracted, intended to be placed in the alveolus after the extraction.

More specifically, the object of the invention is a post-extraction dental implant the objective of which is to increase both the long-term and the short-term stability of the implantation.

### Background of the Invention

Dental implantology techniques are currently widespread, their use and acceptance by both professionals and patients being common. Said techniques allow replacing tooth roots by means of implants to which the corresponding prostheses are in turn coupled.

In early implantology, the extraction of a tooth which was to be replaced by an implant involved waiting about 6-12 months in order to be able to install the fixing. It was accepted that there had to be a wait of that time so that the bone around the extraction area was ossified and was ready to receive and integrate a titanium implant.

However, this protocol prevented responding to the increasingly greater need for quick aesthetic solutions to situations of a traumatic loss of a front tooth due to an accident, periodontal disease or another cause.

Thus, post-extraction implants, which are implanted in the space left by the alveolus, i.e., by the root or roots of the extracted tooth, arose to respond to said need.

These implants have a more pronounced conicity than normal implants since immediately after the extraction takes place the alveolus still maintains its shape and has not closed, therefore it is suitable to adapt to the existing alveolus.

This type of post-extraction implant currently forms parts of a well-known technique which improves the results in aesthetically compromised areas since it conserves bone and gingival tissue and speeds up restoration times, decreasing the psychological trauma due to the dental mutilation and the cost of the restoration.

As has been stated, said implants are structured from elements with a pronounced conicity, the body of which is completely threaded except for its upper part, which defines a non-threaded head.

However, although these known implants fulfill the function for which they have been designed, they have a certain lack of both primary and secondary stability, primary or short-term stability being defined as the stable attachment of the implant to the bone due to a good mechanical anchorage at the time of the insertion, and the secondary or long-term stability being defined as the stable attachment of the implant to the bone due to the growth and remodeling of the bone moths after the insertion.

This instability is mainly due to the fact that since their head is not threaded, they are not secured to the cortical area of the bone, which is the outermost area, whereby gripping capacity and therefore stability is lost.

### Description of the Invention

The post-extraction implant of the invention described below solves the previously mentioned drawbacks because it provides a high primary and secondary stability, generally improving the anchorage to the bone thereof and providing a suitable response to all the requirements demanded for it in each of the steps of its implantation, furthermore being able to be implanted in conditions of little available bone structure and/or considerable closeness of the adjacent teeth.

To that end, the threaded implant of the invention is structured from a body with a general conical geometry in the upper area of which with a larger diameter there is defined a head allowing its use after the extraction, i.e., allowing its insertion in the space left in the bone by the root of a recently extracted tooth.

Furthermore, as an essential element, the implant of the invention has its entire length, also including its upper area or head, threaded, which allows said implant to be screwed not only on the trabecular or inner area of the bone, but also on the cortical or outer area, thus improving the primary stability thereof, i.e., the stability at the time of the insertion.

For the purpose of furthermore improving the retention, the thread is of the self-tapping type, such that said implant itself works the thread in the bone.

On the other hand, for the purpose of improving both the primary and the secondary stability, the implant of the invention includes at least one micro-thread or thread of a smaller size intercalated between the previously described thread, the function of which is based on achieving an even greater fixing to the cortical bone.

Specifically, this additional fixing to the cortical bone is achieved because said bone is more compact than the trabecular bone in which most of the implant is inserted through the larger self-tapping thread, therefore it favors the insertion of these micro-threads, assuring a more solid and long-lasting attachment.

### Description of the Drawings

To complement the description which is being made and for the purpose of aiding to better understand the features of the invention according to a preferred practical embodiment thereof, a set of drawings is attached as an integral part of said description, in which the following has been depicted with an illustrative and non-limiting character:

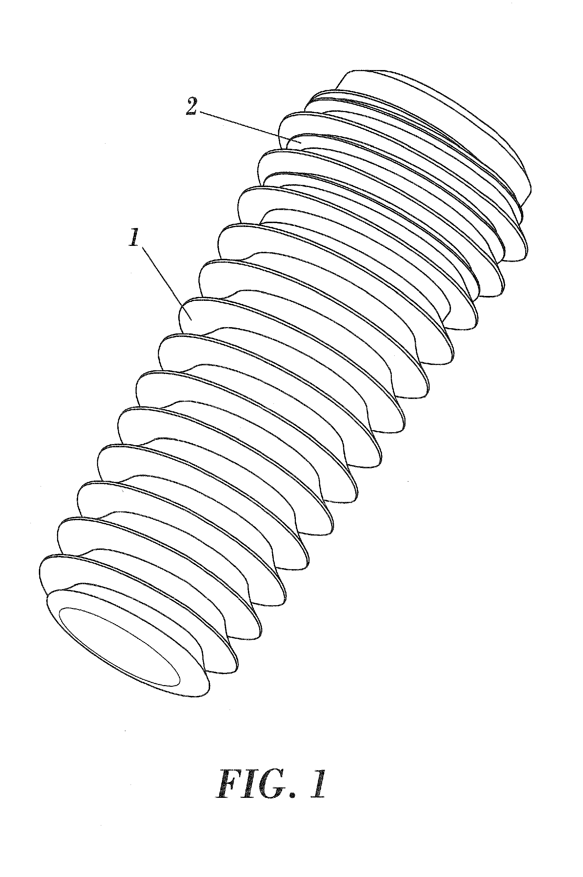

Figure 1 shows a perspective view of the implant of the invention.

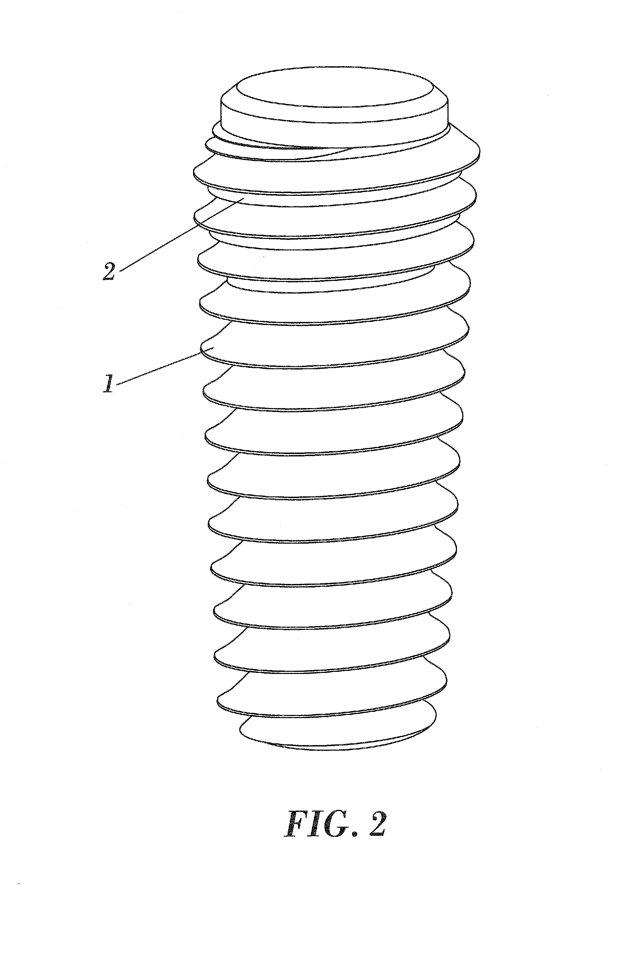

Figure 2 shows a second perspective view of the implant of the invention.

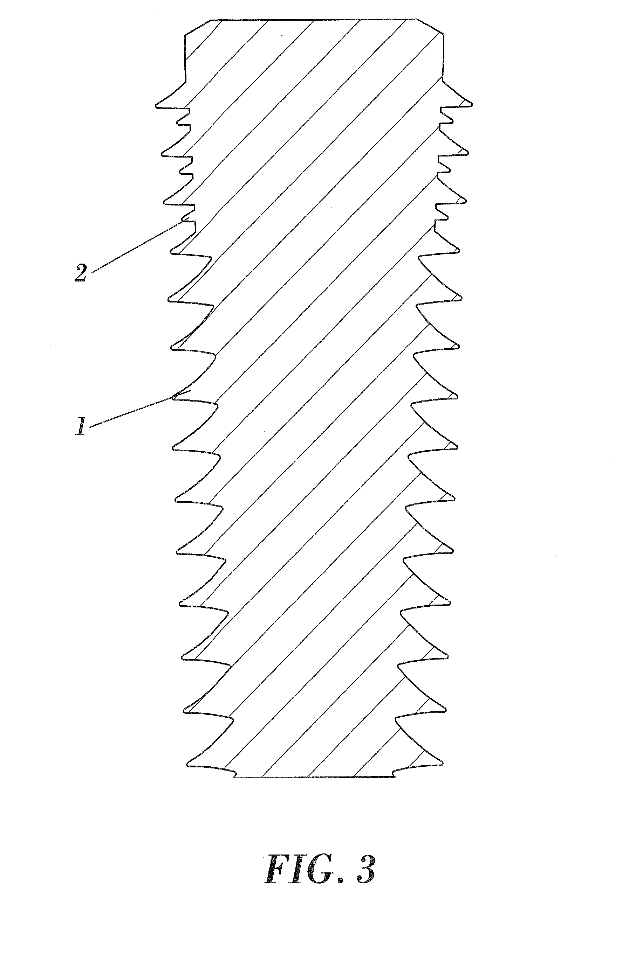

Figure 3 shows a schematized sectioned plan view of the implant of the invention.
Figure 4 shows a section view in which the means for connection to the prosthetic elements according to a possible embodiment of the invention are observed.
Figure 5 shows, finally, a section view in which other means for connection to the prosthetic elements according to another possible embodiment of the invention are shown.

### Preferred Embodiment of the Invention

In view of the figures, an embodiment of the post-extraction implant of the invention can be observed therein which basically comprises a conical body on which there is located a thread (1) extending along its entire length, including the upper area or head, such that the implant can be screwed, through said thread (1), on the cortical area of the bone.

According to a preferred embodiment of the invention, said thread (1) will be of the self-tapping type, thus allowing the implant itself to work the thread on the bone without needing to perform a prior operation.

Furthermore, as can be seen in the figures, for the purpose of achieving a greater fixing to the cortical bone, the post-extraction implant of the invention includes at least one self-tapping micro-thread (2) of a smaller size than the thread (1) and intercalated between it, both the thread (1) and the micro-thread (2) having the same thread pitch which, according to a preferred embodiment of the invention, is between 0.6 and 1 mm.

Furthermore, according to another possible embodiment of the invention, the thread (1) will have a different width in the upper area, of the head, intended to be fixed in the cortical area of the bone, and, preferably of a smaller size than the rest of the thread (1) located along the implant, thus favoring the anchorage.

Thus, according to a possible embodiment of the invention, the thread (1) has a width comprised between 0.3 and 0.5 mm, in which the thread which is located on the body would be comprised between 0.4 and 0.5 mm and the one which is located on the head, of a smaller size according to that stated above, between 0.30-0.35 mm.

On the other hand, the micro-thread (2), also according to a possible practical embodiment of the invention, has a size which is a third of that of the thread of the area of the head (1).

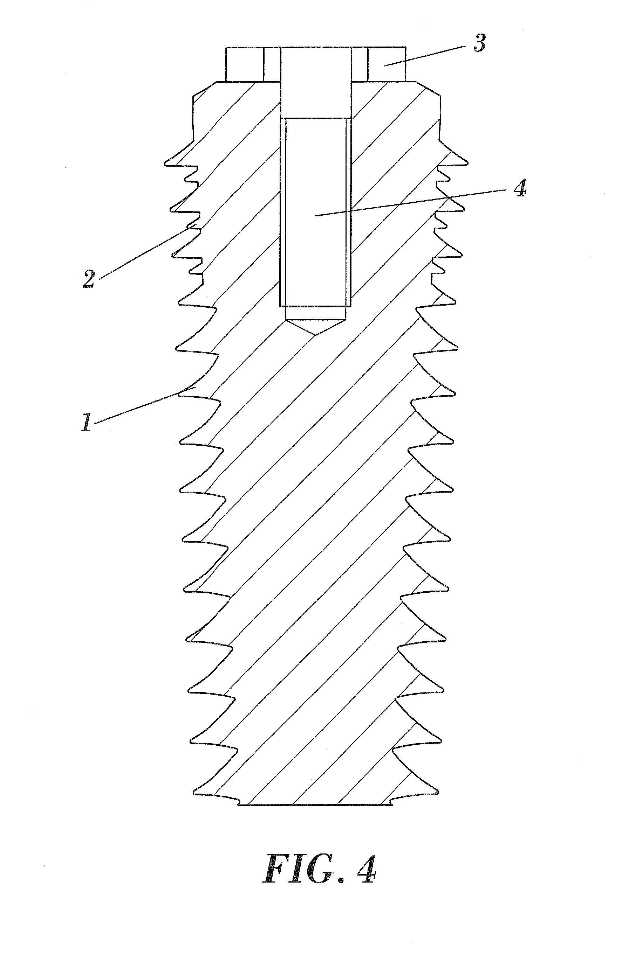

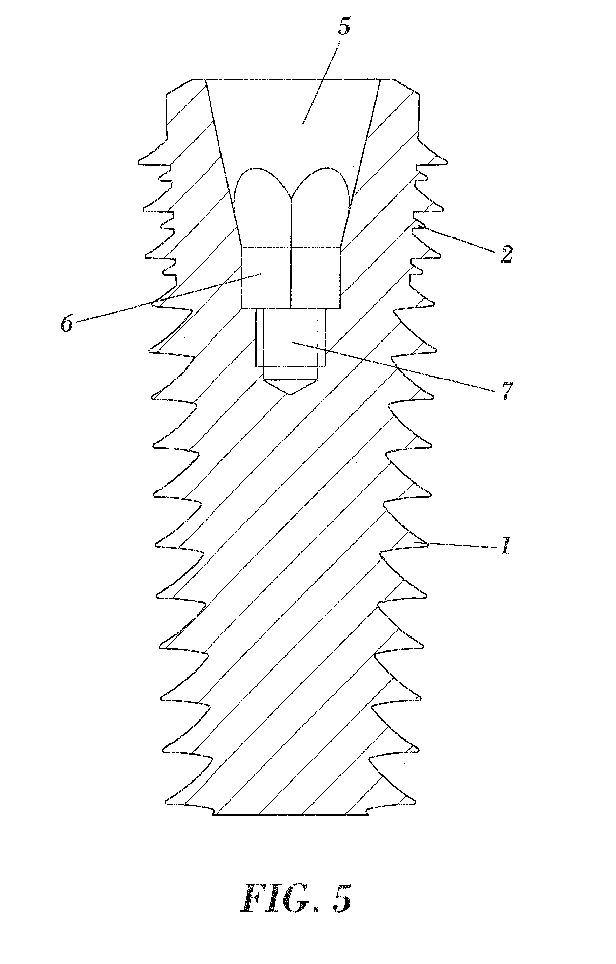

For the purpose of enabling the connection with the prosthetic elements, the post-extraction implant of the invention will have fixing means, as shown in Figures 4 and 5.

More specifically, Figure 4 shows a possible embodiment having an outer connection (3) of a hexagonal type or others, extending at the lower part along the axial axis of the implant by means of a threaded hole (4) for the retention of a threaded element or screw.

Another possible embodiment is shown in Figure 5, in which said fixing means, on this occasion inner fixing means, comprise a conical cavity (5) aligned along the axial axis of the implant and extending at the lower part in a hexagonal cavity (6) below which, and also axially aligned, there is located a threaded hole (7) for the retention of a threaded element or screw.

Thus, in the embodiment shown in said figure 5, the connection will be of the type of those in which the connection between the prosthetic element and the implant will be by means of a conical inner connection under friction with a through bolt, and in which furthermore the hexagonal cavity (6) forms an anti-rotation means.

## Claims

1. Post-extraction dental implant comprising a conical body on which a thread (1) is located, **characterized in that** said thread (1) extends along the entire length of the body such that the screwing of the implant both to the trabecular area and to the cortical area of the bone is assured.

2. Post-extraction dental implant according to claim 1, **characterized in that** it comprises at least one self-tapping micro-thread (2) of a smaller size than the thread (1) and intercalated between it.

3. Post-extraction dental implant according to claim 2, **characterized in that** the thread (1) and the micro-thread (2) have the same thread pitch.

4. Post-extraction dental implant according to claims 1 or 2, **characterized in that** the thread (1) is a self-tapping thread.

5. Post-extraction dental implant according to any of claims 1, 2 or 4, **characterized in that** the thread (1) has a smaller width in the upper area of the implant intended to be fixed in the cortical area of the bone than the width that it has along the rest of the implant.

6. Post-extraction dental implant according to any of the previous claims, **characterized in that** it has means for fixing the prosthetic elements.

7. Post-extraction dental implant according to claim 6, **characterized in that** the fixing means comprise an outer connection (3) extending at the lower part along the axial axis of the implant by means of threaded hole (4) for the retention of a threaded element or screw.

8. Post-extraction dental implant according to claim 7, **characterized in that** the outer connection (3) is hexagonal.

9. Post-extraction dental implant according to claim 6, **characterized in that** the fixing means comprises a conical cavity (5) aligned along the axial axis of the implant and extending at the lower part in a hexagonal cavity (6) below which, and also axially aligned, there is located a threaded hole (7) for the retention of a threaded element or screw.
